# EUROPÄISCHE PATENTSCHRIFT

(11) **EP 1 637 102 B1**
(45) Veröffentlichungstag und Bekanntmachung des Hinweises auf die Patenterteilung: **19.03.2008**
(21) Anmeldenummer: 04405585.3
(22) Anmeldetag: 16.09.2004
(51) Int. Cl.: A61F 9/013, A61B 17/32

(54) **Klinge und dafür geeigneter Klingenträger**
Blade and blade carrying tool therefor.
Lame et son outil porte-lame.

(43) Veröffentlichungstag der Anmeldung: 22.03.2006
(73) Patentinhaber: SIS AG, Surgical Instrument Systems, 2562 Port (CH)
(72) Erfinder: Rathjen, Christian, 28197 Bremen (DE); Hunkeler, Thomas, 3210 Kerzers (CH)
(74) Vertreter: Vogel, Dany

(56) Entgegenhaltungen:
- EP-A- 1 325 721
- EP-A- 1 464 312
- WO-A-20/04056295
- US-A1- 2003 191 484

## Beschreibung

### Technisches Gebiet

Die vorliegende Erfindung betrifft eine Klinge und einen dafür geeigneten Klingenträger. Die vorliegende Erfindung betrifft insbesondere eine Klinge zum Wegschneiden eines Epithellappens von einer Bowmanschen Membran, welche Klinge einen Klingenschaft und eine damit verbundene Klingenspitze mit Schneide umfasst, und einen dafür geeigneten Klingenträger.

### Stand der Technik

Fehlsichtigkeiten wie Myopie (Kurzsichtigkeit), Hyperopie (Weitsichtigkeit oder Übersichtigkeit) oder Astigmatismus (Stabsichtigkeit) können heute durch refraktiv-chirurgische Behandlung dauerhaft korrigiert werden. Refraktiv-chirurgische Behandlungen sind chirurgische Eingriffe am Auge, die die optische Brechkraft des Auges ändern mit dem Ziel, diese einem gewünschten Wert möglichst gut anzunähern. Eines der bedeutendsten Verfahren in der refraktiven Chirurgie ist die so genannte Laser in-situ Keratomileusis (LASIK), bei der das Innere der Hornhaut mit einem computergesteuerten Excimerlaser abgetragen wird, nachdem zuvor ein Hornhautlappen teilweise abgetrennt und weggeklappt wird. Zur Erzeugung des Hautlappens werden typischerweise mechanische Mikrokeratome eingesetzt, bei denen ein angetriebenes Messer den Hautlappen schneidet.

In der Patentanmeldung WO 2004/052254 werden ein Trennelement und ein Verfahren beschrieben, um das sogenannte Epithel der Hornhaut von der darunter liegenden Bowmanschen Membran zu trennen. Das Trennelement umfasst eine Trennschneide, die nicht so scharf ausgebildet ist, dass sie in die Bowmansche Membran eindringt. Nach WO 2004/052254 werden die Epithelzellen durch die Trennschneide weggedrückt, vorzugsweise ohne dabei die interzellulären Bindungen des Epithels zu beschädigen. Die Fäserchen, die das Epithel mit der Bowmanschen Membran verbinden, werden durch die Trennschneide zerteilt, ohne dass dabei in die Bowmansche Membran eingeschnitten wird. Nach WO 2004/052254 weist die Trennschneide eine Dicke auf die wesentlich unter der Dicke des Epithels liegt, vorzugsweise eine Dicke von 15µm.

In der Patentanmeldung US 2003/0018348 wird eine Vorrichtung beschrieben um das Epithel der Hornhaut mechanisch von einem Auge zu trennen. Die Vorrichtung nach US 2003/0018348 umfasst ein Trennelement mit einer Schneide um das Epithel während der Bewegung des Trennelements über das Auge zu entfernen. Nach US 2003/0018348 weist die Schneide eine Dicke auf, die mindestens so dick ist wie eine einzige Epithelzellenschicht, die aber weniger dick ist als die Dicke des Epithels. Vorzugsweise weist die Schneide nach US 2003/0018348 eine Dicke von zwei bis drei Zellschichten des Epithels auf. Nach US 2003/0018348 werden die Epithelzellen während der Bewegung des Trennelements über das Auge weggedrückt, ohne dass dabei die interzellulären Bindungen des Epithels beschädigt werden.

Die bekannten Verfahren und Vorrichtungen zur Entfernung des Epithels von einem Auge weisen den Nachteil auf, dass sie die Bindungen zwischen Bowmanscher Membran und Epithel beim Vorschieben des Trennelements durch Wegdrücken der Epithelzellen trennen. Beim vorwärtsgerichteten Druck wird das Epithel jedoch so verformt, dass Scherkräfte innerhalb des Epithels erzeugt werden, die die Bindungen zwischen den Epithelzellen zerstören können. Beim vorwärtsgerichteten Druck besteht folglich die Gefahr, dass auch bei vorsichtiger Applikation Bindungen zwischen einzelnen Epithelzellen und damit das Epithel, beispielsweise bis zum Zerreissen, zerstört werden, was sich bei der Rekonstruktion der Hornhaut negativ auf die Verheilung des Epitheigewebes mit der Bowmanschen Membran und damit negativ auf den Erfolg der refraktiv-chirurgischen Behandlung auswirken kann

In der Patentanmeldung EP 13257132 wird eine Klinge zum Abtrennen eines Epithellappens beschrieben, welche eine mit einem Klingenschaft verbundene gerundete Klingenspitze mit Schneide aufweist. Die Dicke der Schneide liegt im Bereich von 5 bis 70µm. Die Klinge nach EP 13257132 weist eine glatte Oberfläche auf und ist so ausgebildet, dass sie einen Freiraum zwischen dem Epithel und der Bowmanschen Membran aufspannt.

### Darstellung der Erfindung

Es ist eine Aufgabe der vorliegenden Erfindung eine Klinge zum Wegschneiden eines Epithellappens von einer Bowmanschen Membran und einen dafür geeigneten Klingenträger vorzuschlagen, welche nicht die Nachteile der bekannten Vorrichtungen aufweisen. Es ist insbesondere eine Aufgabe der vorliegenden Erfindung, eine Klinge zum Wegschneiden eines Epithellappens von einer Bowmanschen Membran und einen dafür geeigneten Klingenträger vorzuschlagen, welche ermöglichen die Bindungen zwischen der Bowmanschen Membran und dem Epithel zu lösen, ohne dafür direkten mechanischen Vorschubdruck auf die Epithelzellen ausüben zu müssen.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass bei der Klinge zum Wegschneiden eines Epithellappens von einer Bowmanschen Membran, welche Klinge einen Klingenschaft und eine damit verbundene Klingenspitze mit Schneide umfasst, die Klingenspitze so ausgebildet ist, dass im Zustand, in welchem die Klingenspitze zwischen Epithel und der Bowmanschen Membran eingeführt ist, durch die Klingenspitze ein Freiraum zwischen dem Epithel und der Bowmanschen Membran aufgespannt wird, der eine Vorspannung von Bindungen zwischen dem Epithel und der Bowmanschen Membran bewirkt. Der durch das Einführen der Klingenspitze zwischen Epithel und Bowmanscher Membran erzeugte Freiraum weist einen zur Schneide senkrecht verlaufenden, im Wesentlichen keilförmigen Querschnitt auf, welcher sich von der Scheide hin zu einem Scheitelpunkt verjüngt, an dem das Epithel und die Bowmansche Membran noch miteinander verbunden sind. Die Erzeugung einer Vorspannung auf die Bindungen zwischen dem Epithel und der Bowmanschen Membran hat den Vorteil, dass diese Bindungen nicht durch Vorschubdruck auf die Epithelzellen zerstört werden müssen, so dass das Epithel nicht nachteilig verformt wird und somit interzelluläre Bindungen des Epithels nicht beeinträchtigt werden. Auf Grund der Vorspannung der Bindungen zwischen der Bowmanschen Membran und dem Epithel kann nämlich der Vorschub der Schneide in der Schneideebene so eingestellt werden, dass er gegenüber von lateralen Oszillationsbewegungen der Schneide in der Schneidebene senkrecht zur Vorschubsrichtung vernachlässigbar ist. Bei den lateralen Oszillationsbewegungen wird der bereits abgetrennte Teil des Epithels, der auf der Klingenspitzenoberseite aufliegt, über Reibkräfte mitgeschleppt, was zu einer fortwährenden Wechselbelastung der vorgespannten Bindungen führt. Durch die Wechselbelastung der vorgespannten Bindungen reissen nach und nach Fasern der Bindungen ab, was letztendlich zu einer Art Ermüdungsbruch der Bindung führt.

Vorzugsweise weist die Klinge eine kantenlos und glatt ausgebildete Auflagefläche auf, welche im Zustand, in welchem die Klingenspitze zwischen Epithel und der Bowmanschen Membran eingeführt ist, mindestens teilweise auf die Bowmansche Membran aufdrückt. Durch die kantenlose und glatte Ausführung der Auflagefläche wird erreicht, dass sich die Klinge insbesondere bei lateralen Oszillationsbewegungen in der Schnittebene senkrecht zu der Vorschubsrichtung nicht über einen Abrasionseffekt in die Bowmansche Membran einarbeitet und diese beschädigt. Andererseits wird durch die Auflagefläche Druck auf die Bowmansche Membran ausgeübt und somit die durch den Freiraum bewirkte Vorspannung der Bindungen zwischen Epithel und Bowmanscher Membran aufgebaut respektive stabilisiert.

In einer Ausführungsvariante ist die an der Klingenspitze angeordnete Auflagefläche so von der Unterseite des Klingenschafts abgewinkelt, dass sich für die Klingenspitze ein Freiwinkel ergibt. Durch die Abwinkelung der Auflagefläche von der Unterseite des Klingenschafts ergibt sich für den Klingenschaft bei der Applikation der Klinge ein grösserer Applikationsfreiwinkel als für die Klingenspitze, so dass die Klinge mit (einem Teil) der Auflagefläche der Klingenspitze nicht aber mit dem Klingenschaft auf der Bowmanschen Membran zum Aufliegen kommt. Dadurch werden unnötige zusätzliche Reibkräfte auf der Bowmanschen Membran vermieden und somit einerseits die Bowmansche Membran und andererseits die Antriebsmittel für den Vorschub und die laterale Oszillation der Klinge nicht unnötig belastet.

Vorzugsweise weist die Schneide eine Dicke auf, die im Wesentlichen mindestens der Dicke des Epithels entspricht. Die Schneide weist beispielsweise eine Dicke von 60µm oder mehr auf. Eine Schneidendicke, die mindestens die Grösse der Dicke des Epithels oder eine grössere Dicke aufweist, trägt entscheidend zur Erzeugung des Freiraums zwischen dem Epithel und der Bowmanschen Membran und damit zur Vorspannung der Bindungen zwischen dem Epithel und der Bowmanschen Membran bei. Zudem wird durch die Wahl einer Schneide von dieser Dicke die Gefahr des Einstechens in die Bowmansche Membran weiter vermindert.

Die Schneide ist vorzugsweise gerundet. Vorzugsweise weist die Schneide eine Rundung mit einem Radius von mindestens der Hälfte der Dicke des Epithels oder einen grösseren Radius auf. Der Radius der Rundung der Schneide beträgt beispielsweise 30µm oder mehr. Eine gerundete Schneide respektive Klingenspitze weist den Vorteil auf, dass sie einfach und kostengünstig herstellbar ist, beispielsweise einstückig aus Kunststoff durch ein Spritzgussverfahren. Die gerundete Schneide verringert die Gefahr des Einstechens in die Bowmansche Membran weiter und ist einem eigentlichen Gleiten der Klingenspitze über die Bowmansche Membran förderlich. Die gerundete Schneide respektive Klingenspitze erleichtert zudem die Aufspannung des Freiraums zwischen dem Epithel und der Bowmanschen Membran, da eine gerundete Schneide respektive Klingenspitze mit glatter Oberfläche ohne grosse Reibkräfte zwischen Epithel und Bowmansche Membran einführbar ist, womit auch die Vorspannung der Bindungen zwischen dem Epithel und der Bowmanschen Membran leichter aufgebaut werden kann.

Erfindungsgemäss weist die Klingenspitze eine glatt ausgebildete Klingenspitzenoberseite auf, auf welcher Klingenspitzenoberseite im Zustand, in welchem die Klingenspitze zwischen Epithel und der Bowmanschen Membran eingeführt ist, der Epithellappen aufliegt, und die Klingenspitzenoberseite weist eine Ausnehmung auf, in welcher Ausnehmung eine Steigwand eines Klingenträgers so aufnehmbar ist, dass der Übergang von der Klingenspitzenoberseite zur Steigwand für den beim fortschreitenden Schneiden aufgleitenden Epithellappen kein Hindernis bildet. Eingeführt in den Klingenträger kann somit der weggeschnittene Epithellappen während des Vorschubs der Schneide hindernis- und spannungsfrei über die Klingenspitzenoberseite und über die Steigwand des Klingenträgers weggleiten.

Die vorliegende Erfindung betrifft neben der oben beschriebenen Klinge auch einen Klingenträger, der eingerichtet ist zum entfernbaren Aufnehmen der Klinge. Die oben beschriebene Klinge kann beispielsweise mittels Steckverbindung oder Klemmmechanismus entfernbar mit dem Klingenträger verbunden werden. Der Klingenträger umfasst vorzugsweise bekannte Saugmittel zur Fixierung des Klingenträgers auf einem Auge.

Vorzugsweise umfasst der Klingenträger erste Antriebsmittel, um die Klinge in einer Vorschubsrichtung zu bewegen. Überdies umfasst der Klingenträger zweite Antriebsmittel, um die Schneide der Klinge in einer Schneideebene senkrecht zur Vorschubsrichtung in laterale Oszillationsbewegung zu setzen, wobei eine sich durch die Oszillationsbewegung ergebende kumulierte laterale Weglänge der Schneide wesentlich grösser ist, als eine sich in einem gleichen Zeitraum in der Vorschubsrichtung ergebende Weglänge der Schneide. Zum Beispiel ergibt sich bei einer lateralen Oszillationsbewegung mit einer Frequenz von 150Hz eine maximale laterale Geschwindigkeit der Schneide von ca. 1.5m/sec, was bei einer Vorschubsgeschwindigkeit von 1.5mm/sec eine fast tausendmal grössere kumulierte Weglänge der Schneide in der Lateralen als in der Vorschubsrichtung ergibt. Das heisst im Vergleich mit der Oszillationsbewegung ist der Vorschub vernachlässigbar. Folglich werden die vorgespannten Bindungen zwischen der Bowmanschen Membran und dem Epithel wie oben beschrieben auf Grund der Oszillationsbewegung der Klingenspitze abgebaut, ohne dass dabei die Epithelzellen durch Vorschubdruck beeinträchtigt werden. Die schonende Behandlung der Epithelzellen wirkt sich bei der Rekonstruktion der Hornhaut vorteilhaft auf die Verheilung des Epithelgewebes mit der Bowmanschen Membran aus, was sich wiederum positiv auf den Erfolg der refraktiv-chirurgischen Behandlung auswirkt.

In einer Ausführungsvariante sind die zweiten Antriebsmittel eingerichtet, die laterale Oszillationsbewegung mit einem Oszillationshub der Schneide im Bereich von 2mm bis 4mm, vorzugsweise 3mm, zu erzeugen (Oszillationsamplitude von 1 mm bis 2mm, vorzugsweise ca. 1.5mm).

In einer Ausführungsvariante sind die zweiten Antriebsmittel eingerichtet, die laterale Oszillationsbewegung mit einer Frequenz im Bereich von 150Hz bis 200Hz, vorzugsweise 180Hz, zu erzeugen. Bei dieser Frequenz der Oszillationsbewegung kann eine gute Schnittqualität erreicht werden, mit glatten und unbeschädigten Oberflächen der freigelegten Bowmanschen Membran und der Unterseite des weggeschnittenen Epithellappens.

Vorzugsweise ist der Klingenträger eingerichtet die Klinge so aufzunehmen, dass sich für die Klingenspitze in Bezug zu der Scheideebene ein Applikationsfreiwinkel in einem Bereich von 10° bis 30° ergibt. Der Klingenträger ist beispielsweise eingerichtet die Klinge so aufzunehmen, dass sich für die Klingenspitze in Bezug zu der Scheideebene ein Applikationsfreiwinkel von ca. 15° ergibt. Der gewählte Bereich des Applikationsfreiwinkels, der sich durch die Ausgestaltung der Klingenspitze respektive Klingenschneide und durch den Klingenaufnahmewinkel des Klingenträgers ergibt, ermöglicht auf besonders vorteilhafte Weise die Erzeugung des Freiraums zwischen dem Epithel und der Bowmanschen Membran und damit die Erzeugung der Vorspannung der Bindungen zwischen dem Epithel und der Bowmanschen Membran. Zusätzlich zu den Vorteilen, die sich durch die Dicke der Schneide und durch die Rundung der Schneide ergeben, ermöglicht der Applikationsfreiwinkel der Klingenspitze eine zusätzliche Spreizung des Freiraums und trägt damit weiter zur Erzeugung der Vorspannung bei. Bei der Applikation der Klinge wird durch den Applikationsfreiwinkel der Klingenspitze ein Abheben der Klingenspitze respektive der Schneide von der zu bearbeitenden Bowmanschen Membran verhindert. Dadurch wird ein Zusammenbrechen der Vorspannung verhindert, wenn sich der offene Arbeitswinkel zwischen Klinge und Bowmanscher Membran während der Applikation der Klinge beim Überschreiten des Scheitelpunkts des Auges drastisch verringert.

Der oben beschriebene Klingenträger und eine darin entfernbar angebrachte Klinge, wie oben beschrieben, bilden zusammen eine ophthalmologische Vorrichtung zum Wegschneiden eines Epithellappens von einer Bowmanschen Membran. Vorzugsweise wird zum Wegschneiden eines Epithellappens von der Bowmanschen Membran die Klinge zum Einsetzen in den Klingenträger so selektiert, dass zunächst die Dicke des Epithels gemessen wird, und dass dann eine Klinge gewählt wird, die eine Schneide aufweist mit einer Dicke, die im Wesentlichen mindestens der gemessenen Dicke des Epithels entspricht.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 a zeigt eine Aufsicht einer Klinge zum Wegschneiden eines Epithellappens von einer Bowmanschen Membran, welche Klinge eine Klingenspitze mit Schneide und einen Klingenschaft umfasst.
Figur 1b zeigt einen Längsschnitt der Klinge zum Wegschneiden eines Epithellappens von einer Bowmanschen Membran, welche Klinge eine Klingenspitze mit Schneide und einen Klingenschaft umfasst.
Figur 2a zeigt eine Seitenansicht eines Klingenträgers mit einer darin entfernbar aufgenommenen Klinge beim Wegschneiden eines Epithellappens von einer Bowmanschen Membran.
Figur 2b zeigt eine Detailansicht des Klingenträgers der Figur 2a, in welcher Detailansicht eine in einer Ausnehmung der Klingenspitzenoberseite aufgenommene Steigwand des Klingenträgers gezeigt wird und in welcher Detailansicht ein weggeschnittener Epithellappen gezeigt wird, der auf der Klingenspitzenoberseite und der Steigwand aufliegt.
Figur 3 zeigt einen detaillierten Querschnitt, welcher eine zwischen das Epithel und die Bowmansche Membran eingeführte Klingenspitze illustriert, wobei durch die Klingenspitze ein Freiraum zwischen Epithel und Bowmanscher Membran aufgespannt wird.
Figur 4a zeigt einen Querschnitt, welcher eine Klingenspitze illustriert, die zwischen das Epithel und die Bowmansche Membran eines Auges eingeführt wurde.
Figur 4b zeigt einen Querschnitt, welcher eine Klingenspitze illustriert, die einen Epithellappen bis zum Scheitelpunkt des Auges von der Bowmanschen Membran des Auges weggeschnitten hat.
Figur 4c zeigt einen Querschnitt, welcher eine Klingenspitze illustriert, die einen Epithellappen bis über den Scheitelpunkt des Auges von der Bowmanschen Membran des Auges weggeschnitten hat.

### Wege zur Ausführung der Erfindung

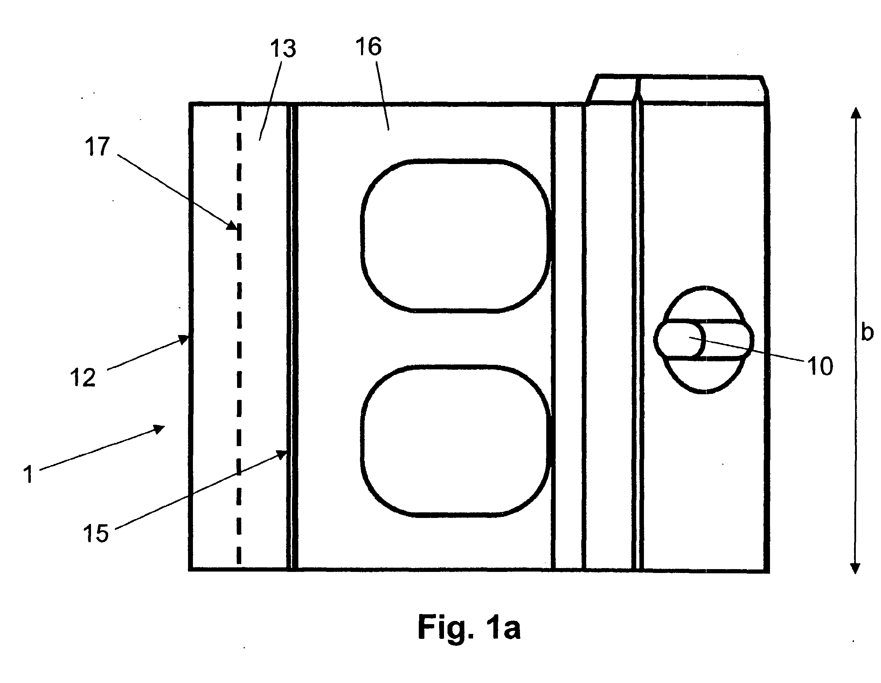

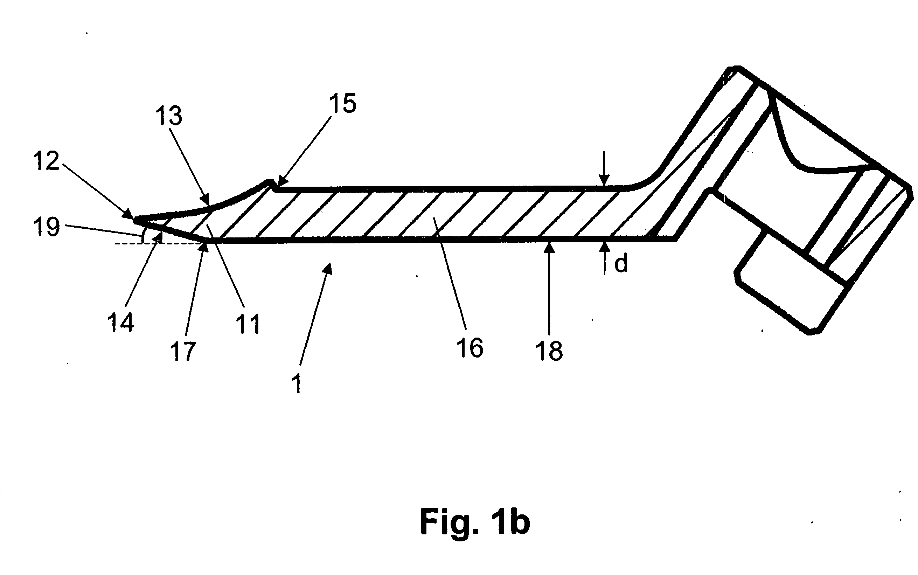

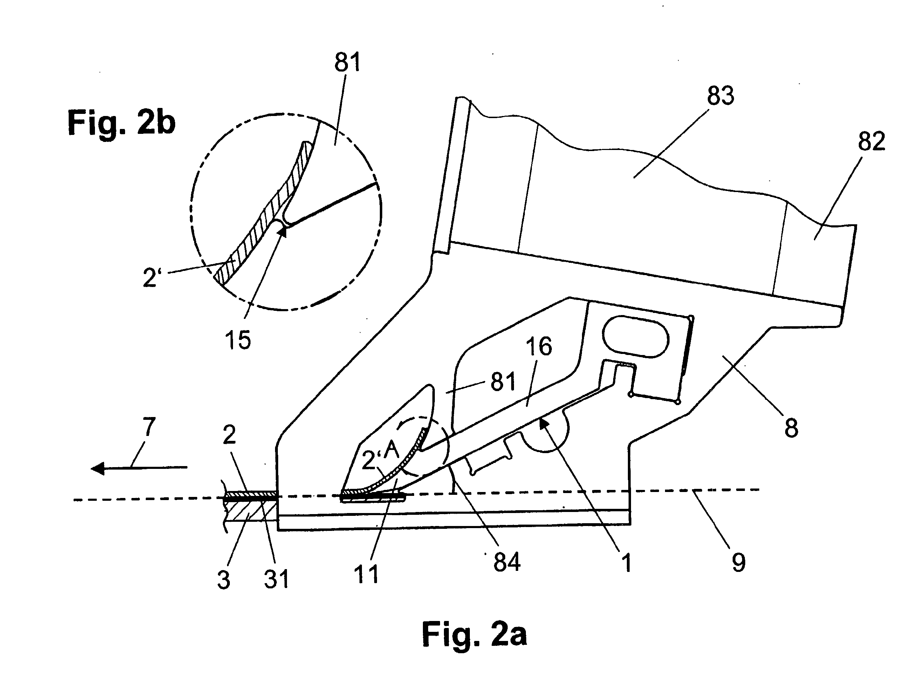

In den Figuren 1a, 1b und 2a bezeichnet das Bezugszeichen 1 eine Klinge zum Wegschneiden eines Epithellappens von einer Bowmanschen Membran. Die Klinge wird vorzugsweise im Spritzgussverfahren einstückig aus Kunststoff hergestellt, beispielsweise aus Polycarbonat. Wie in den den Figuren 1a, 1b und 2a dargestellt ist, umfasst die Klinge 1 eine Klingenspitze 11 und einen Klingenschaft 16. Die Klinge 1 weist eine Breite b im Bereich von 12mm bis 16mm auf. Die Klinge 1 weist eine Dicke d im Bereich von 0.5mm bis 2mm auf. Entlang dem zugespitzten Ende der Klingenspitze 11 erstreckt sich die Schneide 12 der Klinge 1.

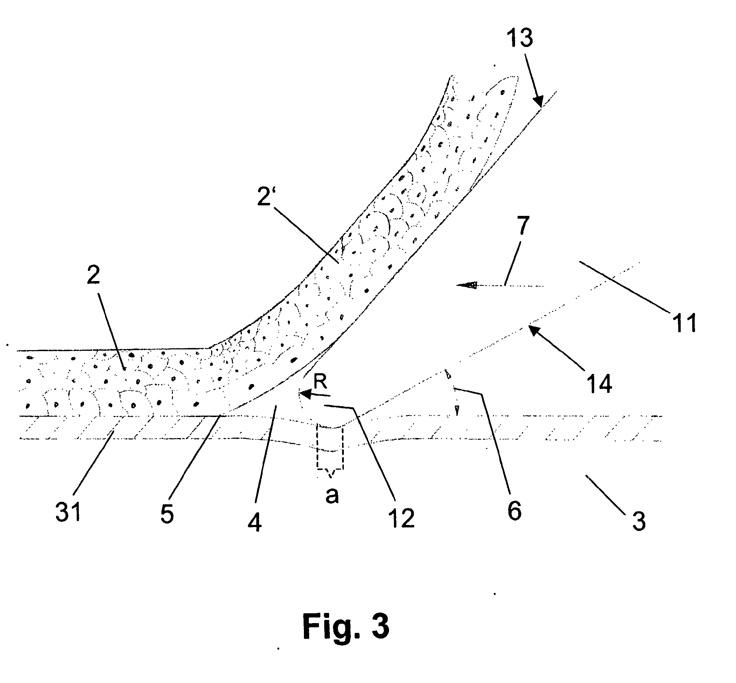

Wie in der Figur 3 dargestellt ist, ist die Schneide 12 vorzugsweise gerundet. Die Rundung der Schneide 12 weist einen Radius R von mindestens der Hälfte der Dicke des Epithels 2 auf. Für die Schneide 12 ergibt sich also mindestens eine Dicke die der Dicke des Epithels 2 entspricht. Der Radius R der Schneide beträgt beispielsweise 30µm oder mehr, was eine Dicke von 60µm oder mehr für die Schneide 12 ergibt. Die bevorzugte Dicke der Schneide 12 liegt im Bereich von einer Dicke des Epithels 2 bis eineinhalb Dicken des Epithels 2, das heisst im Bereiche von ca. 60µm bis 90µm.

Wie in der Figur 1b ersichtlich ist, weist die Klinge 1 eine an der Klingenspitze 11 angeordnete Auflagefläche 14 auf, die kantenlos und glatt ausgebildet ist. Die Auflagefläche 14 ist von der Unterseite 18 des Klingenschafts 16 ab der Kantenlinie 17 abgewinkelt und läuft zur Schneide 12 hin zu, so dass sich für die Klingenspitze 11 ein Freiwinkel 19 von ca. 10° ergibt. Die Klingenspitze 11 weist zudem eine von der Auflagefläche 14 abgewandte, glatt ausgebildete Klingenspitzenoberseite 13 auf, die zur Schneide 12 hin zuläuft. Wie in der Figur 1b ersichtlich ist, bilden die Auflagefläche 14 und die Klingenspitzenoberseite 13 einen sich zur Schneide 12 hin verjüngenden Querschnitt der Klingenspitze 11. Die Länge der Klingenspitze 11 von der Schneide 12 bis zur Kantenlinie 17 beträgt ca. 1 mm bis 2mm.

In der Figur 2a bezieht sich das Bezugszeichen 8 auf einen Klingenträger. Der Klingenträger 8 ist eingerichtet, die Klinge 1 entfernbar aufzunehmen. Für die entfernbare Verbindung der Klinge 1 mit dem Klingenträger 8 umfasst der Klingenträger 8 nicht dargestellte Befestigungsmittel. Die Befestigungsmittel umfassen vorzugsweise eine Steckverbindung. Es sind beispielsweise auch eine Verriegelung, Klemmmittel oder Schraubenmittel möglich. Der Klingenträger 8 umfasst zudem schematisch dargestellte Antriebsmittel 82, 83, beispielsweise Elektromotoren. Die Antriebsmittel 82 sind eingerichtet die Klinge 1 in der Vorschubsrichtung 7 zu bewegen, so dass die Schneide 12 in der Schneideebene 9 in der Vorschubsrichtung 7 bewegt wird. Die Antriebsmittel 83 sind eingerichtet die Schneide 12 der Klinge 1 in der Schneideebene 9 senkrecht zur Vorschubsrichtung 7 in laterale Oszillationsbewegung zu setzen. Die Antriebsmittel 83 sind eingerichtet die laterale Oszillationsbewegung mit einem Oszillationshub der Schneide 12 im Bereich von 2mm bis 4mm (Oszillationsamplitude von 1 mm bis 2mm), vorzugsweise ca. 3mm (Oszillationsamplitude von ca. 1.5mm), und mit einer Frequenz im Bereich von 150Hz bis 200Hz zu erzeugen, vorzugsweise ca. 180Hz. Wie in der Figur 1a dargestellt ist, weist die Klinge eine Ausnehmung 10 auf, in die eine Antriebsnocke der Antriebsmittel 83 zur Übertragung der Oszillationsbewegung aufnehmbar ist.

Der Klingenträger 8 ist eingerichtet, die Klinge 1 so aufzunehmen, dass sich für die Klingenspitze 11 in Bezug zu der Scheideebene 9 ein Applikationsfreiwinkel 6 im Bereich von 10° bis 30° ergibt. Der Applikationsfreiwinkel 6 beträgt vorzugsweise ca. 15°. Der Applikationsfreiwinkel 6 der Klingenspitze 11 ergibt sich aus dem Klingenaufnahmewinkel 84 des Klingenträgers 8 (vorzugsweise 25°) durch Abzug des Freiwinkels 19 der Klingenspitze 11 (vorzugsweise 10°).

Wie in den Figuren 1b und 2a ersichtlich ist, ist die Klingenspitzenoberseite 13 vorzugsweise konkav ausgestaltet. Zudem weist die Klingenspitzenoberseite 13 eine Ausnehmung 15 auf, in welche Ausnehmung 15 eine Steigwand 81 des Klingenträgers 8 aufnehmbar ist. Wie in der Detailsicht der Figur 2b dargestellt ist, führt die konkav ausgestaltete Klingenspitzenoberseite 13 den weggeschnittenen Epithellappen 2' ohne Verklemmung über den Spalt, der zwischen der Klingenspitzenoberseite 13 und der in die Ausnehmung 15 aufgenommenen Steigwand 81 gebildet ist. Zu diesem Zeck ist die Ausnehmung 15 so dimensioniert, dass die Klingenspitzenoberseite 13 beim Übergang zur aufgenommenen Steigwand 81 der Steigwand 81 leicht vorsteht.

Im detaillierten Querschnitt der Figur 3 wird die Klingenspitze 11 im eingeführten Zustand zwischen dem Epithel 2 und der Bowmanschen Membran 31 illustriert. Die Bowmansche Membran 31 befindet sich zwischen dem Epithel 2 und dem Stroma 3. An dieser Stelle soll festgehalten werden, dass sich zwischen dem Epithel 2 und der Bowmanschen Membran 31 zudem eine nicht dargestellte Basalmembran befindet und dass die beschriebene Trennung des Epithels 2 von der Bowmanschen Membran 31 auch eine Trennung des Epithels 2 von dieser Basalmembran einschliessen kann. Wie in der Figur 3 deutlich ersichtlich ist, wird im Zustand, in dem die Klingenspitze 11 zwischen dem Epithel 2 und der Bowmanschen Membran 31 eingeführt ist, von der Klingenspitze 11 der Freiraum 4 zwischen dem Epithel 2 und der Bowmanschen Membran 31 aufgespannt. Der Freiraum 4 bewirkt eine Vorspannung von Bindungen zwischen dem Epithel 2 und der Bowmanschen Membran 31. Die Vorspannung ist am Scheitelpunkt 5, an dem das Epithel 2 und die Bowmansche Membran 31 noch miteinander verbunden sind, am grössten. Wie in der Figur 3 dargestellt ist, liegt die Klingenspitze 11 mit einem an der Schneide 12 liegenden Bereich a der Auflagefläche 14 auf der Bowmanschen Membran 31 auf und übt einen leichten Druck auf die Bowmansche Membran 31 aus. Zudem wird das Epithel 2 durch die Klingenspitzenoberseite 13 abgespreizt. Die Vorspannung der Bindungen wird insbesondere durch den auf die Bowmansche Membran 31 ausgeübten Druck und durch die Abspreizung des Epithels 2 erzeugt.

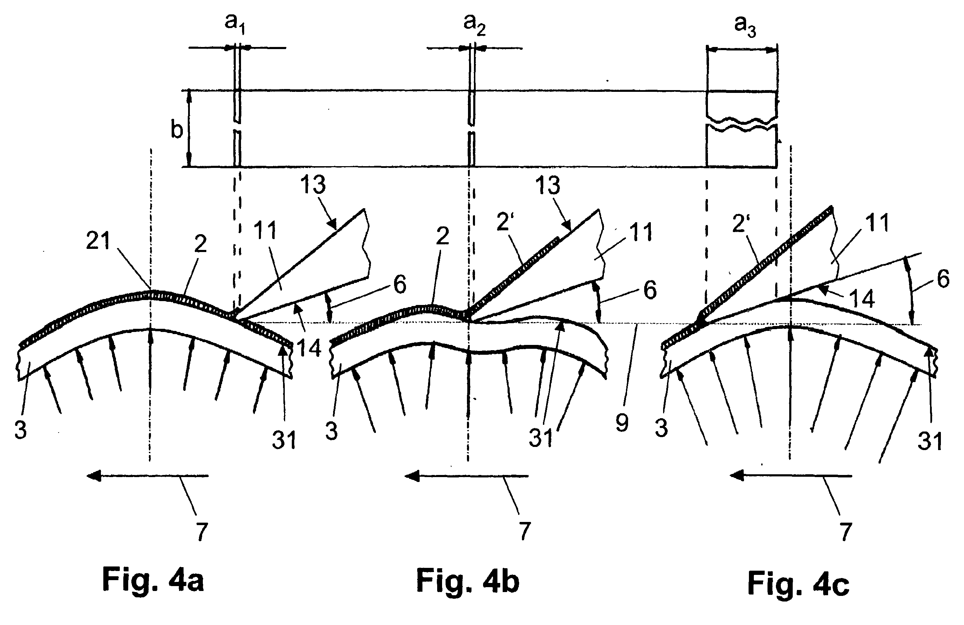

In den Figuren 4a, 4b, 4c werden schematisch verschiedene Phasen beim Wegschneiden des Epithels 2 von der Bowmanschen Membran 31 illustriert. Die Bowmansche Membran 31 ist in den Figuren 4a, 4b, 4c nur schematisch als dünne Schicht über dem Stroma 3 dargestellt. In der Figur 4a wird die Klingenspitze 11 nach dem Durchstechen des Epithels 2 gezeigt, wenn die Klingenspitze 11 zwischen das Epithel 2 und die Bowmansche Membran 31 eingeführt wird. Wie in der Figur 4a gezeigt wird, liegt die Klingenspitze 11 im Bereich a₁ auf der Bowmanschen Membran 31 auf, wie oben mit Bezug zur Figur 3 erläutert wurde. Der angeschnittene Lappen des Epithels 2 gleitet auf die Klingenspitzenoberseite 13 auf. In der Figur 4b wird die Klingenspitze 11 beim Erreichen des Augenscheitelpunkts 21 gezeigt, wenn bereits ungefähr die Hälfte des wegzuschneidenden Epithellappens 2' von der Bowmanschen Membran 31 weggeschnitten wurde. Wie in der Figur 4b gezeigt wird, liegt die Klingenspitze 11 im Bereich a₂ auf der Bowmanschen Membran auf und der weggeschnittene Epithellappen 2' liegt auf der Klingenspitzenoberseite 13 auf und wird durch die Klingenspitze 11 abgespreizt. Die Bereiche a₁ und a₂ entsprechen im Wesentlichen dem Bereich a der Auflagefläche 14, der oben mit Bezug zur Figur 3 beschrieben wurde. In der Figur 4c wird die Klingenspitze 11 nach dem Überschreiten des Augenscheitelpunkts 21 gezeigt, wenn der wegzuschneidende Epithellappen 2' fast in der geplanten Grösse von der Bowmanschen Membran 31 weggeschnitten wurde. Wie in der Figur 4c gezeigt wird, liegt die Klingenspitze 11 im Bereich a₃ auf der Bowmanschen Membran auf. Der Bereich a₃ ist wesentlich grösser als die Bereiche a₁ und a₂ da die Klingenspitze 11 nach dem Überschreiten des Augenscheitelpunkts 21 bei dem in der Figur 4c dargestellten Grenzfall des Applikationsfreiwinkel 6 mit einem immer grösser werdenden Teil der Auflagefläche 14 auf die Bowmansche Membran 31 zu liegen kommt. Vorzugsweise ist der Applikationsfreiwinkel 6 jedoch so gewählt, dass die Klingenspitze 11 auch nach dem Überschreiten des Augenscheitelpunkts 21 nicht mit dem Bereich a₃ der Klingenspitze 11 sondern bloss mit dem an der Schneide 12 liegenden Bereich a (respektive a₁ und a₂) der Auflagefläche 14 auf der Bowmanschen Membran 31 aufliegt. Solange der Applikationsfreiwinkel 6 grösser als 10° ist, wird verhindert, dass die Klingenspitze 11 und insbesondere die Schneide 12 nach dem Überschreiten des Augenscheitelpunkts 21 von der Bowmanschen Membran 31 abhebt. Dadurch kann die Vorspannung der Bindungen zwischen Epithel 2 und Bowmanscher Membran 31 aufrechterhalten werden und der Zusammenbruch des Schneiddrucks verhindert werden.

## Patentansprüche

1. Klinge (1) zum Wegschneiden eines Epithellappens (2') von einer Bowmanschen Membran (31), umfassend einen Klingenschaft (16) und eine damit verbundene Klingenspitze (11) mit Schneide (12), wobei die Klingenspitze (11) so ausgebildet ist, dass im Zustand, in welchem die Klingenspitze (11) zwischen Epithel (2) und der Bowmanschen Membran (31) eingeführt ist, durch die Klingenspitze (11) ein Freiraum (4) zwischen dem Epithel (2) und der Bowmanschen Membran (31) aufgespannt wird, der eine Vorspannung von Bindungen zwischen dem Epithel (2) und der Bowmanschen Membran (31) bewirkt, und wobei die Klingenspitze (11) eine glatt ausgebildete Klingenspitzenoberseite (13) aufweist, auf welcher Klingenspitzenoberseite (13) im Zustand, in welchem die Klingenspitze (11) zwischen Epithel (2) und der Bowmanschen Membran (31) eingeführt ist, der Epithellappen (2') aufliegt, **dadurch gekennzeichnet,**
**dass** die Klingenspitzenoberseite (13) eine Ausnehmung (15) aufweist, in welcher Ausnehmung (15) eine Steigwand (81) eines Klingenträgers (8) so aufnehmbar ist, dass der Übergang von der Klingenspitzenoberseite (13) zur Steigwand (81) für den beim fortschreitenden Schneiden aufgleitenden Epithellappen (2') kein Hindernis bildet.

2. Klinge (1) nach Anspruch 1, **dadurch gekennzeichnet, dass** die Klinge (1) eine kantenlos und glatt ausgebildete Auflagefläche (14) aufweist, welche Auflagefläche (14) im Zustand, in welchem die Klingenspitze (11) zwischen Epithel (2) und der Bowmanschen Membran (31) eingeführt ist, mindestens teilweise auf die Bowmansche Membran (31) aufdrückt.

3. Klinge (1) nach Anspruch 2, **dadurch gekennzeichnet, dass** die Auflagefläche (14) an der Klingenspitze (11) angeordnet ist, wobei die Auflagefläche (14) so von einer Unterseite (18) des Klingenschafts (16) abgewinkelt ist, dass sich für die Klingenspitze (11) ein Freiwinkel (19) ergibt.

4. Klinge (1) nach einem der Ansprüche 1 bis 3, **dadurch gekennzeichnet, dass** die Schneide (12) eine Dicke aufweist, die im Wesentlichen mindestens der Dicke des Epithels (2) entspricht.

5. Klinge (1) nach einem der Ansprüche 1 bis 3, **dadurch gekennzeichnet, dass** die Schneide (12) eine Dicke von mindestens 60µm aufweist.

6. Klinge (1) nach einem der Ansprüche 1 bis 5, **dadurch gekennzeichnet, dass** die Schneide (12) gerundet ist.

7. Klingenträger (8) eingerichtet zum entfernbaren Aufnehmen einer Klinge (1) nach einem der Ansprüche 1 bis 6.

8. Klingenträger (8) nach Anspruch 7, **dadurch gekennzeichnet, dass** der Klingenträger (8) erste Antriebsmittel (82) umfasst um die Klinge (1) in einer Vorschubsrichtung (7) zu bewegen, und dass der Klingenträger (8) zweite Antriebsmittel (83) umfasst um die Schneide (12) der Klinge (1) in einer Schneideebene (9) senkrecht zur Vorschubsrichtung (7) in laterale Oszillationsbewegung zu setzen, wobei eine sich durch die Oszillationsbewegung ergebende kumulierte laterale Weglänge der Schneide (12) wesentlich grösser ist als eine sich in einem gleichen Zeitraum in der Vorschubsrichtung (7) ergebende Weglänge der Schneide (12).

9. Klingenträger (8) nach Anspruch 8, **dadurch gekennzeichnet, dass** die zweiten Antriebsmittel (83) eingerichtet sind, die laterale Oszillationsbewegung mit einem Oszillationshub der Schneide (12) im Bereich von 2mm bis 4mm zu erzeugen.

10. Klingenträger (8) nach einem der Ansprüche 8 oder 9, **dadurch gekennzeichnet, dass** die zweiten Antriebsmittel (83) eingerichtet sind, die laterale Oszillationsbewegung mit einer Frequenz im Bereich von 150Hz bis 200Hz zu erzeugen.

11. Klingenträger (8) nach einem der Ansprüche 7 bis 10, **dadurch gekennzeichnet, dass** der Klingenträger (8) eingerichtet ist die Klinge (1) so aufzunehmen, dass sich für die Klingenspitze (11) in Bezug zu einer Scheideebene (9) ein Applikationsfreiwinkel (6) in einem Bereich von 10° bis 30° ergibt.

12. Klingenträger (8) nach einem der Ansprüche 7 bis 10, **dadurch gekennzeichnet, dass** der Klingenträger (8) eingerichtet ist die Klinge (1) so aufzunehmen, dass sich für die Klingenspitze (11) in Bezug zu einer Scheideebene (9) ein Applikationsfreiwinkel (6) von 15° ergibt.

13. Verfahren zum Selektieren einer Klinge (1) nach einem der Ansprüche 1 bis 6 zum Wegschneiden eines Epithellappens (2') von einer Bowmanschen Membran (31), umfassend Messen der Dicke des Epithels (2) und Selektieren der Klinge (1), die eine Schneide (12) aufweist mit einer Dicke, die im Wesentlichen mindestens der gemessenen Dicke des Epithels (2) entspricht.

## Claims

1. Blade (1) for cutting away an epithelial flap (2') from a Bowman's membrane (31), comprising a blade shaft (16) and, connected to the latter, a blade tip (11) with cutting edge (12), wherein the blade tip (11) is designed in such a way that, in the state in which the blade tip (11) is inserted between epithelium (2) and Bowman's membrane (31), said blade tip (11) spreads open a clearance space (4) between the epithelium (2) and the Bowman's membrane (31), which clearance space (4) results in a pre-tensioning of connections between the epithelium (2) and the Bowman's membrane (31), and wherein the blade tip (11) has a smoothly configured blade tip upper face (13) and, in the state in which the blade tip (11) is inserted between epithelium (2) and Bowman's membrane (31), the epithelial flap (2') lies on this blade tip upper face (13), **characterised in that** the blade tip upper face (13) has a recess (15), in which recess (15) an upward wall (81) of a blade holder (8) can be received in such a way that the transition from the blade tip upper face (13) to the upward wall (81) does not form an obstacle to the epithelial flap (2') sliding up it during continued cutting.

2. Blade (1) according to Claim 1, **characterised in that** the blade (1) has an edgeless and smooth contact surface (14), and, in the state in which the blade tip (11) is inserted between epithelium (2) and Bowman's membrane (31), this contact surface (14) presses at least partially on the Bowman's membrane (31).

3. Blade (1) according to Claim 2, **characterised in that** the contact surface (14) is arranged on the blade tip (11), the contact surface (11) being angled away from an underside (18) of the blade shaft (16) in such a way that the blade tip (11) has a clearance angle (19).

4. Blade (1) according to one of Claims 1 to 3, **characterised in that** the cutting edge (12) has a thickness which corresponds substantially at least to the thickness of the epithelium (2).

5. Blade (1) according to one of Claims 1 to 3, **characterised in that** the cutting edge (12) has a thickness of at least 60 *µ*m.

6. Blade (1) according to one of Claims 1 to 5, **characterised in that** the cutting edge (12) is rounded.

7. Blade (1) holder designed to receive, in a removable manner, a blade (1) according to one of the Claims 1 to 6.

8. Blade holder (8) according to Claim 7, **characterised in that** the blade holder (8) comprises first drive means (82) for moving the blade (1) in an advance direction (7), and **in that** the blade holder (8) comprises second drive means (83) for setting the cutting edge (12) of the blade (1) in a lateral oscillating movement perpendicular to the advance direction in a cutting plane, a cumulative lateral travel of the cutting edge (12) obtained from the oscillating movement being considerably greater than a travel of the cutting edge (12) arising within the same time period in the advance direction (7).

9. Blade holder (8) according to Claim 8, **characterised in that** the second drive means (83) are designed to generate the lateral oscillating movement with an oscillation stroke of the cutting edge (12) in the range of from 2 mm to 4 mm.

10. Blade holder (8) according to one of Claims 8 or 9, **characterised in that** the second drive means (83) are designed to generate the lateral oscillating movement with a frequency in the range of from 150 Hz to 200 Hz.

11. Blade holder (8) according to one of Claims 7 to 10, **characterised in that** the blade holder (8) is designed to receive the blade (1) in such a way that the blade tip (11) has an application clearance angle (6) in a range of from 10° to 30° in relation to a cutting plane (9).

12. Blade holder (8) according to one of Claims 7 to 11, **characterised in that** the blade holder (8) is designed to receive the blade (1) in such a way that the blade tip (11) has an application clearance angle of 15° (6) in relation to a cutting plane (9).

13. Method for selecting a blade (1) according to one of Claims 1 to 6 for cutting away an epithelial flap (2') from a Bowman's membrane (31), comprising measuring the thickness of the epithelium (2) and selecting the blade (1) which has a cutting edge (12) with a thickness corresponding substantially at least to the measured thickness of the epithelium (2).

## Revendications

1. Lame (1) pour découper un lambeau épithélial (2') dans une membrane de Bowman (31), comprenant une tige de lame (16) et une pointe de lame (11) connectée à celle-ci, avec un tranchant (12), la pointe de lame (11) étant réalisée de telle sorte que dans l'état dans lequel la pointe de lame (11) est introduite entre l'épithélium (2) et la membrane de Bowman (31), un espace libre (4) est tendu entre l'épithélium (2) et la membrane de Bowman (31) par la pointe de lame (11), lequel espace libre provoque une précontrainte des liaisons entre l'épithélium (2) et la membrane de Bowman (31), la pointe de lame (11) présentant une face supérieure de pointe de lame (13) lisse, sur laquelle face supérieure de pointe de lame (13), dans l'état dans lequel la pointe de lame (11) est introduite entre l'épithélium (2) et la membrane de Bowman (31), repose le lambeau épithélial (2'),
**caractérisée en ce que**
la face supérieure de pointe de lame (13) présente un évidement (15) dans lequel évidement (15) peut être reçue une paroi montante (81) d'un outil porte-lame (8) de telle sorte que la transition de la face supérieure de pointe de lame (13) à la paroi montante (81) ne forme aucun obstacle au lambeau épithélial (2') glissant dessus lors du découpage continu.

2. Lame (1) selon la revendication 1, **caractérisée en ce que** la lame (1) présente une surface d'appui (14) sans arête et lisse, laquelle surface d'appui (14), dans l'état dans lequel la pointe de lame (11) est introduite entre l'épithélium (2) et la membrane de Bowman (31), presse au moins en partie sur la membrane de Bowman (31).

3. Lame (1) selon la revendication 2, **caractérisée en ce que** la surface d'appui (14) est disposée sur la pointe de lame (11), la surface d'appui (14) étant coudée à l'écart d'une face inférieure (18) de la tige de lame (16) de telle sorte que l'on obtienne un angle libre (19) pour la pointe de lame (11).

4. Lame (1) selon l'une quelconque des revendications 1 à 3, **caractérisée en ce que** le tranchant (12) présente une épaisseur qui correspond essentiellement au moins à l'épaisseur de l'épithélium (2).

5. Lame (1) selon l'une quelconque des revendications 1 à 3, **caractérisée en ce que** le tranchant (12) présente une épaisseur d'au moins 60 *µ*m.

6. Lame (1) selon l'une quelconque des revendications 1 à 5, **caractérisée en ce que** le tranchant (12) est arrondi.

7. Outil porte-lame (8) prévu pour recevoir de manière amovible une lame (1) selon l'une quelconque des revendications 1 à 6.

8. Outil porte-lame (8) selon la revendication 7, **caractérisé en ce que** l'outil porte-lame (8) comprend des premiers moyens d'entraînement (82) pour déplacer la lame (1) dans un sens d'avance (7), et **en ce que** l'outil porte-lame (8) comprend des deuxièmes moyens d'entraînement (83) pour amener le tranchant (12) de la lame (1) en mouvement d'oscillation latéral dans un plan de coupe (9) perpendiculaire au sens d'avance (7), une longueur de course latérale cumulée du tranchant (12) résultant du mouvement d'oscillation étant considérablement plus longue qu'une longueur de course du tranchant (12) parcourue pendant un intervalle identique dans le sens d'avance (7).

9. Outil porte-lame (8) selon la revendication 8, **caractérisé en ce que** les deuxièmes moyens d'entraînement (83) sont prévus pour produire le mouvement d'oscillation latéral avec une course d'oscillation du tranchant (12) dans une plage de 2 mm à 4 mm.

10. Outil porte-lame (8) selon l'une quelconque des revendications 8 ou 9, **caractérisé en ce que** les deuxièmes moyens d'entraînement (83) sont prévus pour produire le mouvement d'oscillation latéral avec une fréquence comprise dans une plage de 150 Hz à 200 Hz.

11. Outil porte-lame (8) selon l'une quelconque des revendications 7 à 10, **caractérisé en ce que** l'outil porte-lame (8) est prévu pour recevoir la lame (1) de telle sorte que l'on obtienne pour la pointe de lame (11) par rapport à un plan de coupe (9) un angle libre d'application (6) compris dans une plage de 10° à 30°.

12. Outil porte-lame (8) selon l'une quelconque des revendications 7 à 10, **caractérisé en ce que** l'outil porte-lame (8) est prévu pour recevoir la lame (1) de telle sorte que l'on obtienne pour la pointe de lame (11) par rapport à un plan de coupe (9) un angle libre d'application (6) de 15°.

13. Procédé pour sélectionner une lame (1) selon l'une quelconque des revendications 1 à 6 pour découper un lambeau épithélial (2') dans une membrane de Bowman (31), comprenant la mesure de l'épaisseur de l'épithélium (2) et la sélection de la lame (1) qui présente un tranchant (12) avec une épaisseur qui correspond essentiellement au moins à l'épaisseur mesurée de l'épithélium (2).
